# EUROPEAN PATENT SPECIFICATION

(11) **EP 0 973 504 B1**
(45) Date of publication and mention of the grant of the patent: **09.03.2005**
(21) Application number: 98903268.5
(22) Date of filing: 30.01.1998
(51) Int. Cl.: A61K 9/12, A61K 31/19, A61K 31/70, A61K 31/715

(54) **HEMOSTATIC AEROSOL COMPOSITION**
BLUTSTILLENDE AEROSOL-ZUBEREITUNG
COMPOSITION D'AEROSOL HEMOSTATIQUE

(30) Priority: 30.01.1997 IE 970062
(43) Date of publication of application: 26.01.2000
(73) Proprietor: Alltracel Development Services Limited, Sallynoggin, Co Dublin (IE)
(72) Inventor: SANTAR, Ivan, 6602 Predklasteri (CZ); KISS, Frantisek, 619 00 Brno (CZ); BRIESTENSKY, Jiri, 503 43 Cernilov (CZ)
(74) Representative: O'Brien, John Augustine
(86) International application number: PCT/IE1998/000003
(87) International publication number: WO 1998/033479

(56) References cited:
- GB-A- 1 254 534
- CHEMICAL ABSTRACTS, vol. 117, no. 1, 4 January 1993 Columbus, Ohio, US; abstract no. 578301, KASPAR: "MICROEMBOLZATION AGENT" XP002062982 & CS 272 002 B (CZECH.) 26 September 1991
- CHEMICAL ABSTRACTS, vol. 106, no. 26, 29 June 1987 Columbus, Ohio, US; abstract no. 219652, BLAZICEK ET AL.: "HEMOSTATIC SUBSTANCE" XP002062983 & CS 235 108 B (CZECH.) 15 February 1987
- CHEMICAL ABSTRACTS, vol. 121, no. 1, 2 January 1995 Columbus, Ohio, US; abstract no. 570118, V. A. STELMAH ET AL.: "POLYCAPRAN. EVALUATION OF HEMOSTATATIC ACTION" XP002062984 & VESTN. BELORUSS. GOS. UNIV., 1993, pages 43-45,
- PATENT ABSTRACTS OF JAPAN vol. 008, no. 236 (C-249), 30 October 1984 & JP 59 116213 A (UNITIKA KK), 5 July 1984,

## Description

The invention relates to a composition of polyanhydroglucuronic acid (PAGA) and its salts. The term polyanhydroglucuronic acid and salts thereof as used herein includes copolymers thereof, especially with anhydroglucose.

### Introduction

Wounds occurring in human and veterinary practice often involve minor lesions such as cuts, excoriations, bums, scalds, and various kinds of open efflorescences be it of eczematous or bacterial origin. Such lesions require rapid covering to arrest bleeding, to disinfect the wound area, and to suppress secondary microbial contamination.

It is widely accepted that the healing essentially consists of three stages which may be simply defined, as: inftammatory/exsudative phase (Phase 1), proliferative phase (Phase 2), differentiation phase (Phase 3).

It is especially important for the healing process to arrest bleeding and to suppress secondary microbial contamination of the wound. Through infiltration of the exsudate, the wound is supplied with leucocytes, neutrophilic granulocytes and monocytes, subsequently being converted to macrophages. Insufficient treatment of even minor lesions may thus give way to massive infections and to considerable complications of the healing process.

The haemostatic effect of oxidised cellulose, introduced into health care practice by pioneering work of Kenyon, Nevell, Rogovin, Jasnitzky and others, has been known since the 1940's. Mechanisms underlying the haernostatic activity of PAGA have been recently explicitly reported e.g. by V A Stelmah et al. (Vestn. Beloruss. Gos. Univ., Ser. 2 (1993), (2) 43-5; cf. CA 1994:570118) to consist in an acceleration of formation of thrombi. The oxidised cellulose is prevailingly manufactured in the form of woven or knitted tissue (such as Surgicel or Interceed by Johnson & Johnson, USA). Its application is therefore mostly limited to specialised institutions such as surgical clinics or ambulances. Also, the existing products have certain contraindications due to their relatively high acidic character. Another disadvantage of the existing products is due to the fact that opening of the sterile packaging outside a sterile area may bring about bacterial contamination of both the sterile product and the wound; the same problem of course applies even to powder form packaged and sterilised in e.g. plastic bottles.

A detailed discussion regarding polyanhydroglucuronic acid and salts thereof is included in our co-pending Patent Application having the same date of filing as this application.

Pressurised aerosol packagings have been available from the 1930's, originally as insect sprays also used during World War II, using chlorofluoroalkanes as the propellant gas. They have been applied to pharmaceutical products since the 1950's, and there has been considerable development towards other types of propellants - less depleting the ozone layer and less contributing to global warming - in the last decades. However, there is no awareness of an aerosol packaged hemostatic of the polyanhydroglucuronic acid type prepared on the basis of oxidised cellulose as yet.

It is therefore the aim of the present invention to provide a product based on polyanhydroglucuronic acid and its salts for use as a sterile absorbable local hemostatic for mass usage by both layman and professional public, enabling easy and rapid application in common wounds and lesions and minimising detrimental effects on the environment.

### Statements of Invention

According to the invention, there is provided a haemostatically active aerosol composition comprising from 0.005 to 0.25 weight parts of microdispersed/microdispersable polyanhydroglucuronic acid and/or salts thereof and from 0.75 to 0.995 weight parts of a suitable dispergating/propellant system wherein the dispergating/propellant system includes liquids which are non-polar or of low polarity and have a surface tension of less than 30 mN/M and a permitivity at 10 kilohertz of less than 10. The term polyanhydroglucuronic acid and salts thereof includes copolymers thereof, especially with anhydroglucose.

The haemostatically active aerosol composition of the invention enables easy and rapid administration and ensures permanent readiness for use, sterility at repeated application, and increased stability due to exclusion of air and UV light access to the product in a pressurised aerosol packaging.

The composition may include at least one pharmaceutical adjuvant which may be selected from one or more substances having suitable anti-microbial, anti-viral, anti-mycotic and/or anti-parasitic effects.

In one embodiment of the invention, the stable microdispersed polyanhydroglucuronic acid and/or salts thereof have the form of particles of 0.1 to 80µm preferably 5 to 15 µm, in size.

In one embodiment of the invention, the propellant used comprises aliphatic and/or alicyclic hydrocarbons with a number of carbon atoms from 1 to 6 and/or aliphatic ethers and/or fluorinated and/or perfluorinated aliphatic hydrocarbons and/or ethers, and/or carbon dioxide and/or nitrogen and/or rare gases and, as the case may be, other auxiliary substances.

The polyanhydroglucuronic acid salts are preferably selected from calcium, magnesium, sodium and/or potassium salts.

The polyanhydroglucuronic acid and/or salts thereof are preferably prepared by a method wherein a polyanhydroglucuronic acid-containing material is subjected to partial or complete hydrolysis and neutralisation in an oxidative environment, the hydrolysate undergoing fractional coagulation to form a stable microdispersed product.

This method provides stable polyanhydroglucuronic acid and salts thereof in essentially a single process carried out in a single vessel.

Preferably the hydrolysis is carried out in an aqueous solution of inorganic and/or organic salts and/or bases. Most preferably the inorganic and/or organic salts and/or bases used for hydrolysis are chlorides, sulphates, carbonates, formates, or acetates of alkali and/or alkaline earth metals, hydroxides of alkali and/or alkaline earth metals, alkylamines, or alkanolamines, in concentrations ranging from 1 x 10⁻³ to 5 mol/l.

In an especially preferred embodiment of the invention the oxidative environment during hydrolysis is established by the presence of agents selected from one or more of hydrogen, sodium or magnesium peroxide, peroxacides and their salts, hypochlorites and chlorites.

Preferably the procedure is carried out at a pH of from 1 to 12, and preferably, at a temperature of from 0 to 100°C.

In a preferred embodiment of the invention the polyanhydroglucuronic acid-containing material is obtained by oxidation of a suitable polysaccharide, such as native or regenerated cellulose or starch.

The polyanhydroglucuronic acid and salts thereof preferably contain in their polymeric chain from 8 to 30 per cent by weight of carboxyl groups, at least 80 per cent by weight of these groups being of the uronic type, at most 5 per cent by weight of carbonyl groups, and at most 0.5 per cent by weight of bound nitrogen.

Preferably the product contains at most 0.2 per cent by weight of bound nitrogen in the polymeric chain.

In a preferred embodiment of the invention the molecular mass of the polymeric chain is from 1x10³ to 3x10⁵ Daltons, most preferably from 5x10³ to 1.5 x 10⁵ Daltons.

The content of the carboxyl groups is in the range of from 12 to 26 per cent by weight and at least 95 per cent of these groups are of the uronic type.

In a particularly preferred embodiment of the invention the product contains at most 1 per cent by weight of carbonyl groups. Typically the carbonyl groups are intra- and intermolecular 2,6 and 3,6 hemiacetals, 2,4-hemialdals and C2 - C3 aldehydes.

Because neutralisation and refining is carried out in a single operation the process is cost effective.

As the product is in a microdispersed form there is enhanced sorption and greater accessibility for blood. Therefore the biological availability is increased and a rapid onset of haemostatis. We have also observed that the product also assists wound healing as a large surface area is presented which is quickly penetrated by body fluids and goes into solution in these fluids. We believe that the product then chemically degrades to achieve more rapid absorption and enhancement of the wound healing process.

The overall homogeneity of the distribution of oxidised groups within the product is increased. Thus. the product has improved reactivity and accessibility to reactive sites for the purpose of binding other substances such as pharmacologically active substances to the product. The average degree of polymerisation is decreased, the distribution of the polymerisation is narrowed and the amount of cellulosic fractions are reduced. This also assists in biodegradation.

The polyanhydroglucuronic acid and salts thereof may be made up of particles sized from 0.1 to 100µm and/or fibres of from 5 to 39µm diameter and up to 30mm length.

Preferably the hydrolysate is let to undergo fractional coagulation by a suitable water-miscible organic solvent, the coagulated product is washed, or dehydrated, using a suitable water-miscible organic solvent, and/or converted, in an appropriate manner, for intended subsequent use.

Preferably the inorganic and/or organic salts and/or bases preferably used for hydrolysis are chlorides, sulphates, carbonates, formates, or acetates or alkali and/or alkaline earth metals, hydroxides of alkali and/or alkaline earth metals, alkylamines, or alkanolamines, in concentrations ranging from 1 x 10³ to 5 mol/l.

Preferably the oxidative environment during hydrolysis is established by the presence of oxidative agents such as hydrogen, sodium or magnesium peroxide, peroxacides and their salts, hypochlorides, or chlorites.

These and other embodiments of the invention are set out in the accompanying claims.

### Detailed Description

The invention will be more clearly understood from the following description thereof given by way of example only.

The essence of the invention consists in the ability of the microdispersed polyanhydroglucuronic acid and salts thereof to form stable dispersions in physiologically indifferent liquids displaying low to zero rate of sedimentation, low viscosity of these colloid-dispersion non-aqueous systems and no tendency to agglomerate at concentrations of 0.5 to 15% b/w.

Of important advantage is the fact that the physicochemical properties of the microdispersed polyanhydroglucuronic acid can be controlled to fit the dispergating liquid or mixture of liquids, thus allowing stable systems suitable as spray fillings to be prepared.

Extensive tests have shown that the microdispersed polyanhydroglucuronic acid and salts thereof prepared by controlled hydrolysis and fractionation, mostly in the form of particles smaller than the size of an erythrocyte, is capable of stimulating the activity of histiocytes and macrophages, which represents another essential advantage of the application of such substances. At the same time, they effectively arrest capillary bleeding of the wound area while getting incorporated into the fibrin net formed. Due to small size of the order of microns, the particles of the microdispersed polyanhydroglucuronic acid and salts thereof undergo, dependent on the chemical composition and physiochemical properties, relatively rapid enzymatic hydrolysis in the wound environment yielding glucose and glucuronic acid, substances inherent to living organism, as final products; in fact, histological observations indicate that they are presumably incorporated into body mucopolysaccharides. Close to neutral pH value of extracts of salts of microdispersed polyanhydroglucuronic acid also substantially contributes to their biocompatibility: no adverse secondary effects due to acidic nature have been reported in their applications.

The presence of reactive carboxyl groups in the microdispersed polyanhydroglucuronic acid and salts thereof is the basis for their ability to chemically bind substances with antibacterial effects such as e.g. derivatives of biguanid, quaternary ammonium salts, or aminosaccharide based antibiotics. Bactericidal activity is also observed for salts or complex salts of certain cations, such as Zn²⁺, Cu²⁺, and to a limited extent Ag⁻, with microdispersed polyanhydroglucuronic acid.

Similarly. we have observed that preparations based on the microdispersed polyanhydroglucuronic acid and salts thereof display certain insecticidal activity. This activity can be enhanced using hydrophobic reactivity of polyanhydroglucuronic acid molecules which allows to anchor. on the powder substance, non-toxic synthetic derivatives of natural pyrethrins such as pyrethroids, notably Permethrin (cis/trans isomer ratio 1:3). Another advantage of compositions according to the invention is thus represented by the possibility to combine, in a single product, hemostatic, bacteriostatic, and insecticidal function. This is important in veterinary medicine for the treatment of both traumatic and artificial lesions in e.g. sheep and cattle, in that it provides a temporary protection against microbial infection and insect attack during healing.

An example of successful combination of an antibiotic and a hemostatic may be represented by the application of neomycine ut sulfas and bacitracinum zincicum bound to a sodium/calcium salt of microdispersed polyanhydroglucuronic acid.

The main problem that had to be solved within the invention concerns the choice of dispergating liquids and propellants to be used in aerosol packaging formulations of the microdispersed polyanhydroglucuronic acid and salts thereof.

Extensive tests have surprisingly revealed that the use of organosols containing several different substituents or highly polar substituents caused the system to easily form coacervates or even to coagulate.

We have found that eg alcoholic dispersions display a relatively low stability with a rapid coagulation and/or sedimentation of particles. The stability is increased with increasing size of the aliphatic chain of the molecule. but the application of higher alcohols is limited from the physiological point of view. We have also found that the hemostatic efficacy of the microdispersed polyanhydroglucuronic acid based products in the initial phase immediately after the spray administration is reduced by the presence of water or polyhydroxycompounds such as glycerol and its derivatives. glycols and polyglycols. Univalent alcohols such as ethanol can induce a stinging pain on application to the wound. Substances of the latter types are therefore preferably avoided in the formulation.

Coagulation and/or sedimentation was surprisingly equally observed in systems where a substance with low polarity has been used, but the molecule contained several different substituents giving rise to an electrostatic non-equilibrium, the Examples of these being dichlorotetrafluoroethane or trichlorofluoromethane. In contrast. low polar substances such as alkanes, C1 to C8 cycloalkanes, or their fluorinated and perfluorinated derivatives, yielded stable dispersion systems with a low sedimentation rate. Examples are methane, ethane, propane, butane, isobutane pentane, 2-methylbutane, 2-methylpropane, 2,2-dimethylpropane and the like. Substances with 3 to 5 carbon atoms such as pentane, neopentane, or a pure petrol fraction free from mercaptanes and aromatics may preferably be used to reduce loss at administration, to improve fixation of the substance upon the treated area.

We have further found that the organic liquid molecule may also contain a heteroatom, preferably oxygen, in the main chain without deteriorating the system stability. Such substances would involve ethers such as dimethylether. diethylether, but also perfluorinated ethers of the methoxy- or ethoxy-nonafluorobutane type.

Extensive tests have shown that the product, though involving an important number of hydrophilic polar groups, can best be dispergated in low polar or non-polar liquids with a low surface tension and low relative permitiviry. In contrast. we have found that liquids with higher polariry and higher surface tension tend to support agglomeration of the product particles and thus to jeopardise the correct function of the aerosol packaging. Besides the effect of microparticles with a large specific surface area. the good dispergability of the microdispersed polyanhydroglucuronic acid and salts thereof may be attributed to their abiliry to enter, in spite of the presence of hydrophilic groups, hydrophobic interactions with the dispergating liquids. The results indicate that stable dispersion systems can be obtained using those of the above substances which display a value of the relative permitivity (dielectric constant at 25°C and 10 kHz) less than 10, preferably less than 5, and that of the surface tension less than 30mN/m, preferably less than 18mN/m. Thus the substances recommended for use involve, preferably, C3 to C5 alkanes, isoalkanes, or cydoalkanes, 1,1,1,2-tetrafluoroethane, dimethylether, methoxy- and ethoxy-nonafluorobutane and mixtures thereof.

Besides the ability to form low sedimenting dispersion systems, the overall criteria limiting the choice of suitable dispergator/propellant systems further include: physiological indifference (low .toxicity, zero or minimum skin and cardiac sensitisation at exposures up to 100000 ppm, no mutagenicity and carcinogenicity, minimum solubility in water and body fluids), indifference in contact with the active substance, high volatility and low heat of evaporation, ability to fix the active substance in the first phase immediately after application on the wound surface, environmental acceptability, and cost.

It is difficult to draw a sharp demarcation line between the dispergating medium suitable for the microdispersed polyanhydroglucuronic acid and salts thereof and the propellant since in some cases both functions can be provided for by one and the same substance such as e.g. n-butane or isobutane. In general, the relevant substances may especially involve:
a) Aliphatic and alicyclic hydrocarbons with 1 to 6 carbon atoms, or aliphatic ethers, notably dimethylether, diethylether, and diisopropylether. While aliphatic hydrocarbons with 1 to 3 carbon atoms could well serve as dispergators for the microdispersed polyanhydroglucuronic acid and salts thereof when under pressure, they evaporate immediately at the output of the spray outlet and thus increase the powder dissipation on spraying and insufficiently fix the powder on the wound surface. It is therefore preferable to use. higher hydrocarbons such as n-butane, isobutane, n-pentane, or isopentane for the given purpose. This group may also include petrolether. pentane/isopentane fraction from petroleum distillation, or a mixture of liquid hydrocarbons currently distributed under the name of medicinal petrol, under the obvious condition of being pure enough from aromatic hydrocarbons and mercaptanes. From the ether group, dimethylether can preferably be used with respect to its suitable physicochemical characteristics.
b) Nonflammable compounds known as fluorohydrocarbons (HFC), perfluorocarbons (PFC), and recently introduced hydrofluoroethers (HFE). Compared to chlorofluorocarbons (CFC), the HFC's, PFC's and HFE's display much reduced life time in the atmosphere and zero to very low ozone-depleting potential (ODP) and global warming potential (GWP). Some may have a slightly increased toxicity and bioreactivity; however, their contact with the wound is very short due to the rapid evaporation rate. The most suitable choice with respect to the properties may be represented by 1,1,1,2-tetrafluoroethane (HFC 134a), or hydrofluoro-ethers such as methoxy-nonafluoroethane (HFE 7100) or 1,1,1,2,3,3-hexafluoro-3-methoxypropane, all of these substances being acceptable from both the physiological and environmental point of view.
   Representatives of both above groups are liquids or substances liquefiable at low pressures (0.2 - 1.4 Mpa) at normal conditions. Further alternatives include:
c) Gaseous substances, which cannot be liquefied at normal conditions, but capable of being absorbed, at least partially, in the powder active substance or in the liquid dispersion system. These include notably carbon dioxide, and nitrous oxide.
d) Gaseous substances not liquefiable at normal conditions and displaying a very limited absorption ability in the liquid dispersion system, such as rare gases, air, and nitrogen.

All of these substances can further be suitably combined with each other to provide for an optimized function of the spray. Based on extensive testing, the preferred combinations include systems such as n-butane or n-pentane/CO₂, medicinal petrol/HFC 134a, isopentane/dimethylether, medicinal petrol/HFE 7100/HFC134a, HFE 7100/CO₂, n-pentane/HFE 7100/N₂.

In summary, the important fact underlying the present invention is that the polyanhydroglucuronic acid and salts thereof create stable dispersion concentrates in liquids that do not compromise the environment, displaying zero or low values of both the ODP and GWP potentials.

An important advantage of the aerosol packaged hemostatic according to the invention consists in the fact that the contents of the packaging can repeatedly be used without the loss of their sterility. The dosing of the active substance can accurately be directed to the wound surface where the powder gets well anchored due to the relatively high speed of incidence of an indifferent dispersion in a liquid that is immiscible with the body fluids and evaporates within a few seconds.

Certain adverse secondary effects are reported for the above listed dispergating and propellant substances, such as weak narcotic effects or skin degreasing on contact for C5 hydrocarbons. However, no such effects have been observed during extensive application tests of the sprays according to the invention because of small applied amounts and short contact time.

An additional specific advantage can be attained when using substances listed under a) above or combinations of substances listed under a) and c) above for preparing the stable dispersions of the micrcdispersed polyanhydroglucuronic acid and salts thereof. Such formulations of the spray allow a simple terminal sterilisation of the finished aerosol packagings to be preformed by gamma radiation.

### EXAMPLES

### Example 1

In this example, the raw material for preparing a salt of microdispersed polyanhydroglucuronic acid were cotton linters containing 99.1 % b/w (by weight) of α-cellulose and oxidised in 60 % nitric acid with an admixture of 3.6 % nitrous acid at a temperature of 28°C in analogy with the procedure of GBP 709684. The resulting product contained:

| | |
|---|---|
| carboxyl groups | 13.7%b/w |
| carbonyl groups | 4.2 % b/w |
| bound nitrogen | 0.48 % b/w |

In a 3000 ml laboratory mixer, 1000 ml of water and 0.158 g of calcium acetate were heated up to 60°C and stirred at 600 rpm. After dissolution of calcium acetate. 2 g of the above defined oxidised cotton linters containing about 8 % of volatile matter were added, temperature increased to 98°C, and the mixture stirred at 2800 rpm for 15 minutes while maintaining the temperature. The temperature was then decreased back to 60°C, pH adjusted to 8.5 by adding sodium hydroxide solution, 25 g of 30% hydrogen peroxide were added. and the hydrolysis continued at the reduced temperature for another 15 minutes. Subsequently the reaction system was cooled down to 40°C, stirring reduced to 300 rpm. and 1300 ml of 92% ethanol were added stepwise during about 10 minutes. The resulting colloid dispersion solution was then filtered, the residue was dispergated into 50% water-ethanol mixture and allowed to stand for one hour. After another filtration the residue was redispergated into 100 ml of isopropanol and allowed to stand for 6 hours. The same procedure was repeated once more, and then the product was filtered and dried in a vacuum drier at a temperature of 40°C.

An analysis of the product obtained yielded:

| | |
|---|---|
| loss on drying | 1.25 % b/w |
| carboxyl group content | 16.8 % b/w |
| carbonyl groups | 0.5 % b/w |
| bound nitrogen content | 0.13 % b/w |
| calcium content | 2.1 % b/w |
| sodium content | 5.2 % b/w |
| particle size | 2 to 5 µm |
| specific surface area | 98 m²/g |
| Molecular weight | 6x10⁴ Daltons |

The product can be used directly as a hemostatic powder or as a component of an aerosol powder spray.

### Example 2

A haemostatic composition in pressurised aerosol packaging has been prepared using stable microdispersed polyanhydroglucuronic acid in the form of calcium/sodium salt as described in Example 1 above. The equipment used included a stainless steel 1000 litre mixer with a propeller stirrer, a stainless steel 30 litre/min metering pump with inner circulation. and an aerosol filling machine (Pamasol type) with one filling head for the dispersion concentrate and two filling heads for the propellant.

The bulk substance used in this example was a calcium/sodium salt of microdispersed polyanhydroglucuronic acid having the following characteristics:

| | |
|---|---|
| particle size 20 - 60 µm | 2 % b/w |
| 10 - 20 µm | 32 % b/w |
| ≤ 10 µm | 66 % b/w |
| specific surface area | 105 m²/g |
| carboxyl group content (total) | 20.2 % b/w |
| carboxyl group content (uronic) | 18.2 % b/w |
| free formaldehyde | 0 % b/w |
| foreign particles | 0 % b/w |
| calcium content | 3.9 % b/w |
| sodium content | 5.6 % b/w |
| bound nitrogen content | 0.02 % b/w |

Chlorohexidine hydrochloride (Ferrosan) in concentration of 0.1 % b/w was added as a bacteriostatic adjuvant. The dispergationf propellant system involved a liquid hydrocarbon mixture (known as medicinal petrol) with density of 652 kg/m³, boiling point 55°C, and residue after evaporation < 2 ppm, and 1.1,1,2-tetrafluoroethane (HFC 134a).

40 kg of the active substance was placed into the mixer. 150 litres of the liquid hydrocarbon mixture added. and the system stirred at 600 rpm for 5 minutes. After addition of 1 kg of chlorohexidine hydrochloride and of another 250 litres of the liquid hydrocarbon mixture, the system was further stirred until a uniform dispersion was obtained. The metering pump was used to dose the dispersion via the filling head of the filling machine into aerosol cans of 80 ml nominal volume in doses of 31 g per can. After inserting a suitable valve. another filling head was used to add 18 g per can of the 1, 1,1,2-tetrafluoroethane propellant.

The finished spray can be used for treatment of bleeding wounds by both the professional or a layman.

### Example 3

The same equipment was used as in Example 2. The active substance consisted of two components, MDOC1 with the same characteristics as in Example 2, and MDOC2 involving a zinc/calcium/sodium salt of microdispersed polyanhydroglucuronic acid having the following properties:

| | |
|---|---|
| carboxyl group content | 19.5 %b/w |
| free formaldehyde | 0 % b/w |
| zinc content | 9.5 % b/w |
| calcium content | 3.9 % b/w |
| sodium content | 5.6 % b/w |
| bound nitrogen content | 0%b/w |

Neomycinum ut sulfas and Bacitracinum zincicum were used as antibacterial adjuvants, n-pentane having density of 625 kg/m³, and boiling point 36°C, as the dispergator, and carbon dioxide (edible grade quality) as the propellant.

38.8 kg of MDOC1 and 1.2 kg of MDOC2 was placed into the mixer together with 0.132 kg of Neomycinum ut sulfas and 0.143 kg (10⁷ IU) of Bacitracinum zincicum, 200 titres of n-pentane added, and the system thoroughly stirred. Another 200 litres of n-pentane were then added and stirred for another 10 minutes. Aerosol cans of 80 ml nominal volume were then filled in doses of 31 g per can. and. after inserting the valves, another filling head was used to pressurise the can by addition of 2 g of compressed carbon dioxide.

The finished spray can be used for professional treatment of bleeding wounds and lesions.

### Example 4

A thoroughly homogenised uniform powder mixture of microdispersed polyanhydroglucuronic acid in the form of magnesium/calcium/sodium and zinc/calcium/sodium salts in the mass ratio of 32: 1 is filled into aerosol cans of 210 ml nominal volume, in doses of 8 g per can on a powder dosing machine (Bosch). Upon closing the can with an appropriate valve, the can is pressurised on the aerosol filling machine by adding 20 g of n-butane and 30 of dimethylether. Finished and gamma sterilised sprays then can be used for treatment of smaller bums or scalds. It can also be applied in e.g. urological or gynecological surgery.

### Example 5

Into the mixer as described in Example 2. 25 kg of calcium/sodium salt of microdispersed polyanhydroglucuronic acid (Example 1). 5.0 kg of calcium stearate. 0.4 kg of chlorohexidine hydrochloride, and 7.7 kg of Permethrin with cis/trans ratio of 25:75 (ICI Plant Protection) are successively placed. After addition of 400 litres of n-pentane and 99 kg of methoxy-nonatluorobucane (HFE 7100), the contents of the mixer are stirred for 15 minutes. The uniform dispersion is then filled into aerosol cans of 210 ml nominal volume in doses of 110 g per can on the filling machine and closed with an appropriate valve. Another ruling head is then used to add 50 g per can of dimethylether.

The finished spray is designed for use in veterinary practice for treatment of wounds and lesions in e.g. sheep and cattle, simultaneously providing a temporary protection against microbial and/or insect attack.

### Example 6

A hydrophilised adduct (MDOC-ACV) of 9-[(2-hydroxy-methoxy)-methyl)-guanin (acyclovir) and a calcium/sodium salt of microdispersed polyanhydroglucuronic acid as described in Example 1, the content of acyclovir in the MDOC-ACV adduct being 50.5 % b/w.

A homogenised mixture of the adduct. the magnesium/ calcium/ sodium salt of microdispersed polyanhydroglucuronic acid according to Example 1. and the zinc/calcium/sodium salt thereof according to Example 2 in the mass ratio of 8.7 : 0.3 : 1.0 is filled into aerosol cans of 120 ml nominal volume in doses of 4 g per can on a powder dosing machine (Bosch). Upon closing the can with an appropriate valve, the aerosol ruling machine is used to pressurise the can by adding 25 g of 2,2-dimethylpropane (neopentane) with a density of 625 kg/m³, and boiling point 9.6°C, on filling head 1, and 23 g of dimethylether with a density of 668 kg/m³.

The finished spray is packed and sterilised by gamma radiation with a dose of 25 kGy and used as a dusting powder in the treatment of e.g. herpes zoster, enabling an easy application and good efficacy even in hairy areas of the body.

## Claims

1. Haemostatically active aerosol composition comprising from 0.005 to 0.25 parts by weight of microdispersed polyanhydroglucuronic acid and/or acceptable salts thereof and from 0.75 to 0.995 parts by weight of a suitable dispergating/propellant system, wherein the dispergating/propellant system includes liquids which are non-polar or of low polarity and have a surface tension of less than 30 mN/M and a permitivity at 10 kilohertz of less than 10 at 25°C.

2. An aerosol composition as claimed in claim 1 wherein the surface tension is less than 18 mN/M.

3. An aerosol composition as claimed in claim 1 or 2 wherein the permitivity at 10 kilohertz is less than 5.

4. An aerosol composition as claimed in any preceding claim wherein the dispergating/propellant system does not include water or polyhydroxy compounds.

5. An aerosol composition as claimed in any preceding claim wherein the system does not include a univalent alcohol.

6. An aerosol composition as claimed in any of claims 1 to 5 wherein the dispergating/propellant system is selected from.one or more of alkanes, C1 to C8 cycloalkanes, fluorinated, or perfluorinated derivatives thereof, preferably the dispergating/propellant system is selected from one or more of methane, ethane, propane, butane, isobutane, pentane, 2-methylbutane, 2-methylpropane, and 2,2-dimethylpropane.

7. An aerosol composition as claimed in claim 6 or 7 wherein the system is selected from one or more of C₃ to C₅ alkanes, cycloalkanes, derivatives thereof and a petrol fraction substantially free from mercaptans and aromatics.

8. An aerosol composition as claimed in any preceding claim wherein the dispergating/propellant system includes a heteroatom in the main chain, preferably the heteroatom is oxygen, preferably the system comprises ethers and perfluorinated derivatives thereof, preferably the system is selected from one or more of dimethylether, diethylether, and perfluorinated ethers, especially methoxy- or ethoxy- nonaflurobutane.

9. An aerosol composition as claimed in any of claims 1 to 8 wherein the system comprises one or more of C₃ to C₅ alkanes, isoalkanes, or cycloalkanes, 1,1,1,2-tetrafluroethane, dimethylether, methoxy- and ethoxy- nonafluorobutane.

10. An aerosol composition as claimed in any preceding claim wherein the system includes one or more aliphatic hydrocarbons, alicylic hydrocarbons of C1 to C6, aliphatic ethers or mixtures thereof, preferably the aliphatic hydrocarbons are selected from those having from 4 to 6 carbon atoms, preferably the aliphatic ethers are selected from dimethylether, diethylether and diisopropylether, most preferably the ether is dimethylether.

11. An aerosol composition as claimed in any preceding claim wherein the system includes one or more hydrofluorocarbons, perfluorocarbons or hydrofluroethers.

12. An aerosol composition as claimed in claim 11 wherein the system includes one or more hydrofluorocarbons or hydrofluoroethers, preferably the hydrofluorocarbon is 1,1,1,2-tetrafluoroethane (HFC 134a), or the hydrofluoroethers are selected from methoxy nonafluoroethane (HFE7100) and 1, 1, 1,2,3,3- hexafluoro-3-methoxypropane.

13. An aerosol composition as claimed in any preceding claim wherein the system includes a gaseous substance which is not liquifiable at normal aerosol operating conditions.

14. An aerosol composition as claimed in claim 13 wherein the gaseous substances are at least partially absorbable by the microdispersed polyanhydroglucuronic acid and/or salts thereof, preferably the gaseous substance is selected from either carbon dioxide and/or nitrous oxide, or the gaseous substance has limited absorbability in the system and is preferably selected from one or more of rare gases, air and nitrogen.

15. An aerosol composition as claimed in any preceding claim wherein the system includes n-butane and carbon dioxide.

16. An aerosol composition as claimed in any of claims 1 to 14 wherein the system includes n-pentane and carbon dioxide.

17. An aerosol composition as claimed in any of claims 1 to 14 wherein the system includes medicinal petrol and HFC 134a.

18. An aerosol composition as claimed in any of claims 1 to 14 wherein the system includes isopentane and dimethylether.

19. An aerosol composition as claimed in any of claims 1 to 14 wherein the system includes medicinal petrol, HFE 7100 and HFC 134a.

20. An aerosol composition as claimed in any of claims 1 to 14 wherein the system includes HFE 7100 and CO₂.

21. An aerosol composition as claimed in any of claims 1 to 14 wherein the system includes n-pentane, HFE 7100 and N₂.

22. An aerosol composition as claimed in any preceding claim wherein the composition includes at least one pharmaceutically acceptable adjuvant, preferably the adjuvant is selected from one or more substances having suitable anti-microbial, anti-viral, anti-mycotic and/or anti-parasitic effects.

23. An aerosol composition as claimed in any preceding claim wherein the stable microdispersed polyanhydroglucuronic acid and/or salts thereof are in the form of particles having a size from 0.1 to 80 µm, preferably the stable microdispersed polyanhydroglucuronic acid and/or salts thereof are in the form of particles having a size of from 5 to 15 µm.

24. An aerosol composition as claimed in any preceding claim wherein the polyanhydroglucuronic salts are selected from calcium, magnesium, sodium and/or potassium salts.

25. An aerosol composition as claimed in any preceding claim wherein the microdispersed polyanhydroglucuronic acid and salts thereof contain in their polymeric chain from 8 to 30 per cent by weight of carboxyl groups, at least 80 per cent by weight of these groups being of the uronic type, at most 5 per cent by weight of carbonyl groups, and at most 0.5 per cent by weight of bound nitrogen.

26. An aerosol composition as claimed in claim 25 wherein the polyanhydroglucuronic acid and salts thereof contain in their polymeric chain at most 0.2 per cent by weight of bound nitrogen, preferably the polyanhydroglucuronic acid and salts thereof have a molecular mass of the polymeric chain of from 1x10³ to 3x10⁵ Daltons, preferably the polyanhydroglucuronic acid and salts thereof have a molecular mass of the polymeric chain of from 5x10³ to 1.5 x 10⁵ Daltons, preferably the content of carboxyl groups is in the range of from 12 to 26 per cent by weight, at least 95 per cent of these groups being of the uronic type, preferably the polymeric chain contains at most I per cent by weight of carbonyl groups, preferably the carbonyl groups are intra- and intermolecular 2,6 and 3,6 hemiacetals, 2,4- hemialdals and C2-C3 aldehydes.

27. An aerosol composition as claimed in any preceding claim wherein the polyanhydroglucuronic acid and/or salts thereof are prepared by a method wherein a polyanhydroglucuronic acid-containing material is subjected to partial or complete hydrolysis and neutralisation in an oxidative environment, the hydrolysate undergoing fractional coagulation to form a stable microdispersed product.

28. An aerosol composition as claimed in claim 27 wherein the hydrolysis is carried out in an aqueous solution of inorganic and/or organic salts and/or bases, preferably the inorganic and/or organic salts and/or bases used for hydrolysis are chlorides, sulphates, carbonates, formates, or acetates of alkali and/or alkaline earth metals, hydroxides of alkali and/or alkaline earth metals, alkylamines, or alkanolamines, in concentrations ranging from 1 x 10⁻³ to 5 mol/l, preferably the oxidative environment during hydrolysis is established by the presence of agents selected from one or more of hydrogen, sodium or magnesium peroxide, peroxacides and their salts, hypochlorites and chlorites, preferably the hydrolysate undergoes fractional distillation by a suitable water-miscible organic solvent, preferably the coagulated product is washed, or dehydrated, using a suitable water-miscible organic solvent, and/or converted, in an appropriate manner, preferably the procedure is carried out at a pH of from 1 to 12, preferably the procedure is carried out at a temperature of from 0 to 100°C.

29. An aerosol composition as claimed in claims 27 or 28 wherein the polyanhydroglucuronic acid-containing material is obtained by oxidation of a suitable polysaccharide, such as native or regenerated cellulose or starch.

## Patentansprüche

1. Hämostatisch aktive Aerosol-Zusammensetzung umfassend von 0,005 bis 0,25 Gewichtsteile mikrodispergierte Polyanhydroglucuronsäure und/oder verträgliche Salze davon und von 0,75 bis 0,995 Gewichtsteile eines geeigneten Dispergier-/Treibmittel-Systems, worin das Dispergier-/Treibmittel-System Flüssigkeiten einschließt, die nicht polar oder von geringer Polarität sind und eine Oberflächenspannung von weniger als 30 mN/m und eine Permittivität bei 10 Kilohertz von weniger als 10 bei 25 °C aufweisen.

2. Aerosol-Zusammensetzung nach Anspruch 1, worin die Oberflächenspannung weniger als 18 mN/m beträgt.

3. Aerosol-Zusammensetzung nach Anspruch 1 oder 2, worin die Permittivität bei 10 Kilohertz weniger als 5 beträgt.

4. Aerosol-Zusammensetzung nach einem der vorangehenden Ansprüche, worin das Dispergier-/Treibmittel-System kein(e) Wasser oder Polyhydroxy-Verbindungen einschließt.

5. Aerosol-Zusammensetzung nach einem der vorangehenden Ansprüche, worin das System keinen einwertigen Alkohol einschließt..

6. Aerosol-Zusammensetzung nach einem der Ansprüche 1 bis 5, worin das Dispergier-/Treibmittel-System aus einem oder mehr von Alkanen, C₁- bis C₈-Cycloalkanen, fluorierten oder perfluorierten Derivaten davon ausgewählt ist, wobei das Dispergier-/Treibmittel-System bevorzugt aus einem oder mehr von Methan, Ethan, Propan, Butan, Isobutan, Pentan, 2-Methylbutan, 2-Methylpropan und 2,2-Dimethylpropan ausgewählt ist.

7. Aerosol-Zusammensetzung nach Anspruch 6 oder 7, worin das System aus einem oder mehr von C₃- bis C₅-Alkanen, Cycloalkanen, Derivaten davon und einer Benzinfraktion, die weitgehend frei von Mercaptanen und Aromaten ist, ausgewählt ist.

8. Aerosol-Zusammensetzung nach einem der vorangehenden Ansprüche, worin das Dispergier-/Treibmittel-System ein Heteroatom in der Hauptkette einschließt, wobei das Heteroatom bevorzugt Sauerstoff darstellt, wobei das System bevorzugt Ether und perfluorierte Derivate davon umfasst, wobei das System bevorzugt aus einem oder mehr von Dimethylether, Diethylether und perfluorierten Ether, besonders Methoxy- oder Ethoxynonafluorbutan ausgewählt ist.

9. Aerosol-Zusammensetzung nach einem der Ansprüche 1 bis 8, worin das System eines oder mehr von C₃- bis C₅-Alkanen, -Isoalkanen oder -Cycloalkanen, 1,1,1,2-Tetrafluorethan, Dimethylether, Methoxy- und Ethoxynonfluorbutan umfasst.

10. Aerosol-Zusammensetzung nach einem der vorangehenden Ansprüche, worin das System einen oder mehr aliphatische(n) Kohlenwasserstoff(e), alicycliscfne(n) Kohlenwasserstoff(e) auf C₁ bis C₆, aliphatischen Ethern oder Gemische davon einschließt, wobei die aliphatischen Kohlenwasserstoffe bevorzugt aus denen ausgewählt sind, die von 4 bis 6 Kohlenstoffatome aufweisen, wobei die aliphatischen Ether bevorzugt aus Dimethylether, Diethylether und Diisopropylether ausgewählt sind, wobei der Ether am bevorzugtesten Dimethylether darstellt.

11. Aerosol-Zusammensetzung nach einem der vorangehenden Ansprüche, worin das System einen oder mehr Fluorkohlenwasserstoff(e), Perfluorkohlenstoff(e) oder Hydrofluorether einschließt.

12. Aerosol-Zusammensetzung nach Anspruch 11, worin das System einen oder mehr Fluorkohlenwasserstoff(e) oder Hydrofluorether einschließt, wobei der Fluorkohlenwasserstoff bevorzugt 1,1,1,2-Tetrafluorethan (HFC 134a) darstellt, oder die Hydrofluorether aus Methoxynonafluroethan (HFE 7100) und 1,1,1,2,3,3-Hexafluor-3-methoxypropan ausgewählt sind.

13. Aerosol-Zusammensetzung nach einem der vorangehenden Ansprüche, worin das System eine gasförmige Substanz einschließt, die bei normalen Aerosol-Betriebsbedingungen nicht verflüssigbar ist.

14. Aerosol-Zusammensetzung nach Anspruch 13, worin die gasförmigen Substanzen mindestens teilweise durch die mikrodispergierte Polyanhydroglucuronsäure und/oder Salze davon absorbierbar sind, wobei die gasförmige Substanz bevorzugt aus entweder Kohlendioxid und/oder Distickstoffoxid ausgewählt ist oder die gasförmige Substanz im System eine begrenzte Absorbierbarkeit aufweist und bevorzugt aus einem oder mehr Edelgasen, Luft oder Stickstoff ausgewählt ist.

15. Aerosol-Zusammensetzung nach einem der vorangehenden Ansprüche, worin das System n-Butan und Kohlendioxid einschließt.

16. Aerosol-Zusammensetzung nach einem der Ansprüche 1 bis 14, worin das System n-Pentan und Kohlendioxid einschließt.

17. Aerosol-Zusammensetzung nach einem der Ansprüche 1 bis 14, worin das System medizinisches Benzin und HFC 134a einschließt.

18. Aerosol-Zusammensetzung nach einem der Ansprüche 1 bis 14, worin das System Isopentan und Dimethylether einschließt.

19. Aerosol-Zusammensetzung nach einem der Ansprüche 1 bis 14, worin das System medizinisches Benzin, HFE 7100 und HFC 134a einschließt.

20. Aerosol-Zusammensetzung nach einem der Ansprüche 1 bis 14, worin das System HFE 7100 und CO₂ einschließt.

21. Aerosol-Zusammensetzung nach einem der Ansprüche 1 bis 14, worin das System n-Pentan, HFE 7100 und N₂ einschließt.

22. Aerosol-Zusammensetzung nach einem der vorangehenden Ansprüche, worin die Zusammensetzung mindestens ein pharmazeutisch verträgliches Adjuvans einschließt, wobei das Adjuvans bevorzugt aus einer oder mehr Substanz(en) mit geeigneten antimikrobiellen, antiviralen, antimykotischen und/oder antiparasitären Wirkungen ausgewählt ist.

23. Aerosol-Zusammensetzung nach einem der vorangehenden Ansprüche, worin die stabile mikrodispergierte Polyanhydroglucuronsäure und/oder Salze davon in der Form von Partikeln mit einer Größe von 0,1 bis 80 µm vorliegt, wobei die stabile mikrodispergierte Polyanhydroglucuronsäure und/oder Salze davon bevorzugt in der Form von Partikeln, die eine Größe von 5 bis 15 µm aufweisen, vorliegen.

24. Aerosol-Zusammensetzung nach einem der vorangehenden Ansprüche, worin die Salze der Polyanhydroglucuronsäure aus Calcium-, Magnesium-, Natriumund/oder Kaliumsalzen ausgewählt sind.

25. Aerosol-Zusammensetzung nach einem der vorangehenden Ansprüche, worin die mikrodispergierte Polyanhydroglucuronsäure und Salze davon in ihrer polymeren Kette von 8 bis 30 Gew.-% Carboxylgruppen enthalten, wobei mindestens 80 Gew.-% dieser Gruppen vom Uronsäuretyp, höchstens 5 Gew.-% Carbonylgruppen und höchstens 0,5 Gew.-% gebundener Stickstoff sind.

26. Aerosol-Zusammensetzung nach Anspruch 25, worin die Polyanhydroglucuronsäure und Salze davon in ihrer Polymerkette höchstens 0,2 Gew.-% gebundenen Stickstoff enthalten, wobei die Polyanhydroglucuronsäure und Salze davon bevorzugt eine Molekularmasse der Polymerkette von 1 x 10³ bis 3 x 10⁵ Dalton aufweisen, wobei die Polyanhydroglucuronsäure und Salze davon bevorzugt eine Molekularmasse der Polymerkette von 5 x 10³ bis 1,5 x 10⁵ Dalton aufweisen, wobei der Gehalt von Carboxylgruppen bevorzugt im Bereich von 12 bis 26 Gew.-% liegt, wobei mindestens 95 % dieser Gruppen vom Uronsäuretyp sind, wobei die Polymerkette bevorzugt höchstens 1 Gew.-% Carbonylgruppen enthält, wobei die Carbonylgruppen bevorzugt intra- und intermolkulare 2,6- und 3,6-Halbacetale, 2,4-Halbacetale und C₂-C₃-Aldehyde darstellen.

27. Aerosol-Zusammensetzung nach einem der vorangehenden Ansprüche, worin die Polyanhydroglucuronsäure und/oder Salze davon anhand eines Verfahrens hergestellt werden, worin ein Polyanhydroglucuronsäure enthaltendes Material teilweise oder vollständig der Hydrolyse und Neutralisation in einem oxidativen Milieu ausgesetzt wird, wobei das Hydrolysat zur Bildung eines stabilen mikrodispergierten Produkts der fraktionierten Koagulation unterzogen wird.

28. Aerosol-Zusammensetzung nach Anspruch 27, worin die Hydrolyse in einer wässrigen Lösung von anorganischen und/oder organischen Salzen und/oder Basen durchgeführt wird, wobei die für die Hydrolyse verwendeten anorganischen und/oder organischen Salze und/oder Basen bevorzugt Chloride, Sulfate, Carbonate, Formiate oder Acetate von Alkali- und/oder Erdalkalimetallen, Hydroxide von Alkali- und/oder Erdalkalimetallen, Alkylaminen oder Alkanolaminen in Konzentrationen darstellen, die im Bereich von 1 x 10⁻³ bis 5 mol/l liegen, wobei das oxidative Milieu bevorzugt während der Hydrolyse durch die Anwesenheit von Mitteln etabliert wird, die aus einem oder mehr von Sauerstoff, Natrium- oder Magnesiumperoxid, Peroxosäuren und ihren Salzen, Hypochloriten und Chloriten ausgewählt sind, wobei das Hydrolysat bevorzugt der fraktionierten Destillation mithilfe eines geeigneten mit Wasser mischbaren organischen Lösungsmittels unterzogen wird, wobei das koagulierte Produkt unter Verwendung eines geeigneten mit Wasser mischbaren organischen Lösungsmittels bevorzugt gewaschen oder dehydratisiert und/oder auf eine angemessene Weise umgewandelt wird, wobei das Verfahren bevorzugt bei einem pH von 1 bis 12 durchgeführt wird, wobei das Verfahren bevorzugt bei einer Temperatur von 0 bis 100 °C durchgeführt wird.

29. Aerosol-Zusammensetzung nach Anspruch 27 oder 28, worin das Polyanhydroglucuronsäure enthaltende Material durch Oxidation eines geeigneten Polysaccharids, wie zum Beispiel nativer oder regenerierter Cellulose oder Stärke erhalten wird.

## Revendications

1. Composition aérosol hémostatiquement active comprenant de 0,005 à 0,25 parties en poids d'acide polyanhydroglucuronique microdispersé et/ou ses sels acceptables et de 0,75 à 0,995 parties en poids d'un système de dispergation/propulsion approprié, dans laquelle le système de dispergation/propulsion inclut des liquides qui sont non polaires ou de faible polarité et ont une tension superficielle de moins de 30 mN/m et une permittivité à 10 kilohertz de moins de 10 à 25°C.

2. Composition aérosol selon la revendication 1, dans laquelle la tension superficielle est de moins de 18 mN/m.

3. Composition aérosol selon la revendication 1 ou 2, dans laquelle la permittivité à 10 kilohertz est de moins de 5.

4. Composition aérosol selon l'une quelconque des revendications précédentes, dans laquelle le système de dispergation/propulsion n'inclut pas d'eau ou de composés polyhydroxy.

5. Composition aérosol selon l'une quelconque des revendications précédentes, dans laquelle le système n'inclut aucun alcool univalent.

6. Composition aérosol selon l'une quelconque des revendications 1 à 5, dans laquelle le système de dispergation/propulsion est choisi parmi un ou plusieurs alcanes, cycloalcanes en C1 à C8 et leurs dérivés fluorés ou perfluorés, le système de dispergation/propulsion étant de préférence choisi parmi un ou plusieurs groupes méthane, éthane, propane, butane, isobutane, pentane, 2-méthylbutane, 2-méthylpropane et 2,2-diméthylpropane.

7. Composition aérosol selon la revendication 6 ou 7, dans laquelle le système est choisi parmi un ou plusieurs alcanes ou cycloalcanes en C₃ à C₅, leurs dérivés et une fraction essence principalement dépourvue de mercaptans et de constituants aromatiques.

8. Composition aérosol selon l'une quelconque des revendications précédentes, dans laquelle le système de dispergation/propulsion inclut un hétéroatome dans la chaîne principale, l'hétéroatome étant de préférence un atome d'oxygène, le système comprenant de préférence des éthers et leurs dérivés perfluorés, le système étant de préférence choisi parmi un ou plusieurs groupes diméthyléther, diéthyléther et éthers perfluorés, particulièrement le méthoxy- et l'éthoxynonafluorobutane.

9. Composition aérosol selon l'une quelconque des revendications 1 à 8, dans laquelle le système comprend un ou plusieurs alcanes, isoalcanes ou cycloalcanes en C₃ à C₅, groupes 1,1,1,2-tétrafluroéthane, diméthyléther, méthoxy- et éthoxy-nonafluorobutane.

10. Composition aérosol selon l'une quelconque des revendications précédentes, dans laquelle le système inclut un ou plusieurs hydrocarbures aliphatiques, hydrocarbures alicyliques en C1 à C6, éthers aliphatiques ou leurs mélanges, les hydrocarbures aliphatiques étant de préférence choisis parmi ceux ayant 4 à 6 atomes de carbone, les éthers aliphatiques étant de préférence choisis parmi les groupes diméthyléther, diéthyléther et diisopropyléther, l'éther étant le plus préférentiellement le diméthyléther.

11. Composition aérosol selon l'une quelconque des revendications précédentes, dans laquelle le système inclut un ou plusieurs hydrofluorocarbures, perfluorocarbures ou hydrofluoroéthers.

12. Composition aérosol selon la revendication 11, dans laquelle le système inclut un ou plusieurs hydrofluorocarbures ou hydrofluoroéthers, l'hydrofluorocarbure étant de préférence 1,1,1,2-tétrafluoraéthane (HFC 134a), ou les hydrofluoroéthers étant de préférence choisis parmi le méthoxynonafluoroéthane (HFE7100) et 1,1,1,2,3,3-hexafluoro-3-méthoxypropane.

13. Composition aérosol selon l'une quelconque des revendications précédentes, dans laquelle le système inclut une substance gazeuse qui n'est pas liquéfiable dans les conditions d'utilisation d'un aérosol normales.

14. Composition aérosol selon la revendication 13, dans laquelle les substances gazeuses sont au moins partiellement absorbables par l'acide polyanhydroglucuronique microdispersé et/ou ses sels, la substance gazeuse étant de préférence choisie parmi le dioxyde de carbone et/ou l'oxyde nitreux, ou la substance gazeuse a une absorbabilité limitée dans le système et est de préférence choisie parmi un ou plusieurs gaz rares, l'air et l'azote.

15. Composition aérosol selon l'une quelconque des revendications précédentes, dans laquelle le système inclut du n-butane et du dioxyde de carbone.

16. Composition aérosol selon l'une quelconque des revendications 1 à 14, dans laquelle le système inclut du n-pentane et du dioxyde de carbone.

17. Composition aérosol selon l'une quelconque des revendications 1 à 14, dans laquelle le système inclut une essence médicamenteuse et du HFC 134a.

18. Composition aérosol selon l'une quelconque des revendications 1 à 14, dans laquelle le système inclut de l'isopentane et du diméthyléther.

19. Composition aérosol selon l'une quelconque des revendications 1 à 14, dans laquelle le système inclut une essence médicamenteuse, du HFE7100 et du HFC 134a.

20. Composition aérosol selon l'une quelconque des revendications 1 à 14, dans laquelle le système inclut du HFE7100 et du CO₂.

21. Composition aérosol selon l'une quelconque des revendications 1 à 14, dans laquelle le système inclut du n-pentane, du HFE7100 et du N₂.

22. Composition aérosol selon l'une quelconque des revendications précédentes, dans laquelle la composition inclut au moins un additif pharmaceutiquement acceptable, l'additif étant de préférence choisi parmi une ou plusieurs substances ayant des effets antimicrobiens, antiviraux, antimycotiques et/ou antiparasitaires appropriés.

23. Composition aérosol selon l'une quelconque des revendications précédentes, dans laquelle l'acide polyanhydroglucuronique microdispersé stable et/ou ses sels sont sous la forme de particules ayant une taille de 0,1 à 80 µm, l'acide polyanhydroglucuronique microdispersé stable et/ou ses sels étant de préférence sous la forme de particules ayant une taille de 5 à 15 µm.

24. Composition aérosol selon l'une quelconque des revendications précédentes, dans laquelle les sels polyanhydroglucuroniques sont choisis parmi des sels de calcium, de magnésium, de sodium et/ou de potassium.

25. Composition aérosol selon l'une quelconque des revendications précédentes, dans laquelle l'acide polyanhydroglucuronique microdispersé et ses sels contiennent dans leur chaîne polymère de 8 à 30 pour cent en poids de groupes carboxyle, au moins 80 pour cent en poids de ces groupes étant de type uronique, au plus 5 pour cent en poids de groupes carbonyle et au plus 0,5 pour cent en poids d'azote lié.

26. Composition aérosol selon la revendication 25, dans laquelle l'acide polyanhydroglucuronique et ses sels contiennent dans leur chaîne polymère au plus 0,2 pour cent en poids d'azote lié, l'acide polyanhydroglucuronique et ses sels ayant de préférence une chaîne polymère de masse moléculaire de 1x10³ à 3x10⁵ Daltons, l'acide polyanhydroglucuronique et ses sels ayant de préférence une chaîne polymère de masse moléculaire de 5x10³ à 1,5x10⁵ Daltons, la teneur en groupes carboxyle étant de préférence de l'ordre de 12 à 26 pour cent en poids, au moins 95 pour cent de ces groupes étant de type uronique, la chaîne polymère contenant de préférence au moins 1 pour cent en poids de groupes carbonyle, les groupes carbonyle étant de préférence des 2,6- et 3,6-hémiacétaux, 2,4-hémialdaux et aldéhydes en C2-C3 intra- et intermoléculaires.

27. Composition aérosol selon l'une quelconque des revendications précédentes, dans laquelle l'acide polyanhydroglucuronique et/ou ses sels sont préparés selon un procédé dans lequel une matière contenant de l'acide polyanhydroglucuronique est soumise à une hydrolyse partielle ou complète et à une neutralisation dans un environnement oxydant, l'hydrolysat subissant une coagulation fractionnée pour former un produit microdispersé stable.

28. Composition aérosol selon la revendication 27, dans laquelle l'hydrolyse est effectuée dans une solution aqueuse de sels minéraux et/ou organiques et/ou de bases minérales et/ou organiques, les sels minéraux et/ou organiques et/ou bases minérales et/ou organiques utilisés pour l'hydrolyse étant de préférence des chlorures, sulfates, carbonates, formiates ou acétates de métaux alcalins et/ou alcalino-terreux, hydroxydes de métaux alcalins et/ou alcalino-terreux, alkylamines ou alkanolamines dans des concentrations de 1 x 10⁻³ à 5 mol/l, l'environnement oxydant pendant l'hydrolyse étant de préférence établi par la présence d'agents choisis parmi un ou plusieurs peroxydes d'hydrogène, de sodium ou de magnésium, peroxoacides et leurs sels, hypochlorites et chlorites, l'hydrolysat subissant de préférence une distillation fractionnée à l'aide d'un solvant organique miscible dans l'eau approprié, le produit coagulé étant de préférence lavé, ou déshydraté, à l'aide d'un solvant organique miscible dans l'eau approprié et/ou converti de manière appropriée, le procédé étant de préférence effectué à un pH de 1 à 12, le procédé étant de préférence effectué à une température de 0 à 100°C.

29. Composition aérosol selon les revendications 27 ou 28, dans laquelle la matière contenant l'acide polyanhydroglucuronique est obtenue par oxydation d'un polysaccharide approprié, tel que la cellulose ou l'amidon natifs ou régénérés.
